# EUROPEAN PATENT SPECIFICATION

(11) **EP 2 708 741 B1**
(45) Date of publication and mention of the grant of the patent: **15.04.2015**
(21) Application number: 12184018.5
(22) Date of filing: 12.09.2012
(51) Int. Cl.: F03D 11/00, F16C 19/52, G01N 27/92, G01N 33/30

(54) **Method and arrangement to monitor a bearing of a wind turbine**
Verfahren und Anordnung zur Überwachung eines Lagers einer Windturbine
Procédé et dispositif pour surveiller un palier d'une éolienne

(43) Date of publication of application: 19.03.2014
(73) Proprietor: Siemens Aktiengesellschaft, 80333 München (DE)
(72) Inventor: Nieuwenhuizen, John, 8700 Horsens (DK)

(56) References cited:
- EP-A1- 0 060 588
- EP-A1- 2 484 900
- EP-A2- 0 288 940

## Description

The invention describes a method and an arrangement to monitor the condition of a bearing in a wind turbine.

A wind turbine transfers the energy of moving air into electrical energy. The wind turbine transfers the energy of the wind into the rotation of a rotor arrangement. The rotor arrangement comprises one or more rotor blades that are connected to a hub.

The rotation of the rotor arrangement is transferred to the rotor of an electrical generator. The electrical generator transforms the rotational energy of the rotor arrangement into electrical energy.

There are mainly two concepts of wind turbines. The first concept transforms the rotational speed of the rotor arrangement via a gear. The rotational speed is multiplied before the rotation is transferred to the electrical generator.

Another concept transfers the rotation of the rotor arrangement to the electrical generator without the use of a gearbox. These wind turbines are called gearless or direct driven wind turbines.

Bearings are widely used in wind turbines. In geared wind turbines the rotor arrangement and the main shaft is supported by bearings. Bearings are used in the gearbox and in the electrical generator. In addition bearings are used in the pitch drive to change the blade angle of the rotor blades and in the yaw system.

In a direct driven wind turbine the rotor arrangement and the rotor of the electrical generator are mostly supported by one main bearing. This main bearing has to be rigid enough to withstand the loads present in the rotating part of the wind turbine for a long time.

The exchange of such a main bearing is difficult and results in high costs for the parts and the replacement and results in a long down-time of the wind turbine. Thus an exchange of the main bearing has to be avoided as long as possible.

A bearing comprises an inner and an outer shell, one shell is a rotating part and the other shell is a stationary part. The outer shell as well as the inner shell of the bearing can be defined as the rotating part of the bearing; this depends on the construction of the wind turbine.

The bearing can be a rolling element bearing comprising rollers or balls between the two bearing shells. The bearing can also be a sliding bearing, a hydrodynamic bearing or any other kind of bearing.

To keep the main bearing in a good working condition, it is important to avoid possible damages to the bearing. Damages can occur by insufficient lubrication, foreign particles in the bearing or sparking in the bearing due to lightning or static discharges. These causes lead to a weaker lubrication and/or a higher wear in the bearing damaging the shells or the rolling elements in the bearing.

Several methods and arrangement are known to avoid damage at the main bearing coming from lightning.

WO2011/069686 A1 describes a lightning protection system for a wind turbine having an electrically grounded structure part, a main shaft, electrically and mechanically connected therewith. A blade hub is rotatively connected via a main bearing to the main shaft and blades connected to the blade hub. It comprises a down-conductor attachable inside a blade, a high voltage conductor for guiding lightning current, wherein a first end of the high voltage conductor is in electrical communication with the down-conductor. It further comprises a high voltage contact attachable inside the main shaft, wherein a second end of the high voltage conductor is in electrical communication with the high voltage contact and wherein a lightning current is guided from the down-conductor via the high voltage conductor to the high voltage contact.

WO2011/084723 A2 describes an assembly, system and method for discharging a rotating shaft encompassed by a stationary housing. The system includes an annular charge-dissipating component and a biasing element oriented to apply an axial force to the charge-dissipating component, holding it in contact with the shaft and the housing while the shaft is rotating. The biasing element may be a coil spring or a wave spring. A shaft grounding upgrade kit includes a conductive ring member and a cylindrical spring having a diameter that is the same as that of the conductive ring member. The spring constant of the cylindrical spring is such that the spring holds the conductive ring member within the housing. The conductive ring member may be constructed of gray iron and the cylindrical spring is one of a wave spring and a coil spring.

WO 1984/001673 A1 discloses an overvoltage protector in an apparatus having two moving parts. The parts are in mutual communication via a bearing, one part being stationary in relation to a substructure at an electrical earth potential. The moving part can be subjected to a voltage and the stationary part is electrically connected to the earthed substructure. For preventing a current passing through the bearing between the parts, an electrical insulator is provided in the current path which can occur through the bearing as a result of the different potentials of the parts. The moving part has a projection at a distance from a means connected to electrical earth such that a spark discharge gap is formed between mutually moving parts. The bearings are protected against current by the electrical insulator up to a given voltage threshold. In order that this voltage threshold will not be exceeded, the spark discharge gap comes into operation at a lower value than this voltage threshold and keeps the voltage difference between the parts at this lower value.

JP 2005151749 A discloses a thunderbolt protective device for the bearing of an aerogenerator. It has a switching controller which provides a switching command to switching driver to switch electric rod to non-contact and contact states with respect to bearing housing. An electric rod has free-end contact which contacts or non-contacts a bearing housing to supply electricity to an outer-ring and inner-ring. A switching controller provides switching command to a switching driver to switch the electric rod to non-contact and contact states.

In addition to avoid damages in the bearing it is important to monitor the condition of the bearing, to detect possible problems in time to avoid further damages in the bearing.

It is known to detect micro cracks in a bearing by using ultrasonic excitation and sensors. The temperature of the bearing can be controlled using temperature sensors. The condition information is gathered and transferred to a control site. The condition is analyzed and the need for maintenance or repair determined.

US2011/0020122 A1 discloses a computer implemented method that includes receiving condition information from a plurality of condition detection sensors coupled to a wind turbine and receiving wind turbine controller information. An anomaly detection algorithm is applied to identify maintenance activities for the wind turbine as a function of both the wind turbine condition information and the wind turbine controller information.

US2011/0125419 A1 discloses a system and method that may provide a relatively low cost, relatively robust data acquisition and analysis system useful for condition-based maintenance. The system may be useful for condition-based maintenance in industrial applications, e.g., of equipment and/or machinery. The industrial monitoring system may be used, for example, to monitor a condition of rotating machinery. e.g. a wind turbine. The system and method may include data analysis that may be useful for anticipating a need for maintenance, repair and/or replacement of one or more components.

The bearings of a wind turbine, especially the main bearing of a direct driven wind turbine, are crucial parts in the wind turbine. The monitoring of the condition of a bearing is thus very important.

Monitoring the condition of a bearing of a wind turbine should be done continuously during the operation of the wind turbine.

EP 2 484 900 discloses an example of a monitored bearing.

Maintenance can be arranged in time, when the analysis of the monitored condition of a bearing of the wind turbine, indicates that maintenance is necessary.

To achieve this, a good and continuous monitoring of the condition of the bearing is necessary.

The aim of the invention is therefore to provide an improved method and arrangement to monitor the condition of a bearing.

The aim is reached by the features of the independent claims. Preferred embodiments of the invention are described in the dependent claims.

A bearing of a wind turbine comprises a first bearing shell and a second bearing shell and a layer of lubrication between the first and the second bearing shell.

A Method to measure the condition of the lubrication in the bearing comprises the steps of applying a voltage from a voltage source to the bearing shells, so that an electrical potential is building up between the bearing shells. The level of the voltage applied to the bearing shells is increased until the break down voltage of the layer of lubrication is reached.

The voltage applied to the bearing shells is measured to detect and measure the value of the break-down voltage of the layer of lubrication in the bearing.

A bearing of a wind turbine can be a rolling element bearing or a kind of sliding bearing. Bearings are used in a wind turbine as pitch bearings at the rotor blades, as main bearings to support the rotating drive train or as a yaw bearing to support the nacelle on the tower for example.

A bearing comprises two bearing shells and at least one layer of lubrication between the bearing shells. The layer of lubrication is important to reduce the friction and the wear in the bearing. The layer of lubrication needs to be in a good condition without discontinuities or gaps to fulfill its purpose.

The layer of lubrication needs to be monitored to make sure that the condition of the layer of lubrication is good and the friction and wear in the bearing is minimized.

A voltage source is connected to the bearing shells so that an electric potential can be applied between the bearing shells. The voltage source is variable so that the level of the voltage can be varied. The voltage is increased to build up a growing potential between the bearing shells.

The layer of lubrication is insulating up to a certain voltage. At a certain border voltage the electric potential between the bearing shells is discharging over the layer of lubrication. This voltage level is called the break down voltage.

The break down voltage is measured. After the discharge the electrical potential is build up again between the bearing shells. When the potential is again high enough, the potential is discharging again and the break down voltage can be detected again.

When the layer of lubrication is thick and the condition of the lubrication material is good, the break down voltage is high. When the layer of lubrication is thin or the material used for lubrication is in a bad condition, the break down voltage is low.

Thus the level of the break down voltage indicates whether the lubrication is good enough to protect the bearing properly.

The break down voltage can be measured repeatedly during the operation of the bearing. It is not necessary to stop the turbine or to open the bearing to control the condition of the lubrication and the bearing.

Thus the condition of the layer of lubrication can be controlled virtually continuously. Thus the wind turbine does not need to be stopped to control the condition of the layer of lubrication. Thus the operating time of the wind turbine is increased. Thus the amount of energy produced with the wind turbine is increased. Thus the costs of energy produced are reduced.

The value of the break down voltage is stored in a memory.

Thus the measurement value of the break down voltage is available for further use.

More then one measured value can be stored in the memory. Thus the development of a row of measurements can be observed. Thus the development of the condition of the layer of lubrication can be investigated.

The measured value of the break down voltage is compared to a reference value.

A certain bearing construction with a certain lubrication and a certain thickness of the layer of lubrication shows a certain predetermined break down voltage. This value of the break down voltage is used as a reference value. This is the value that can be expected in good conditions of the layer of lubrication.

Thus the difference between the measured value of the break down voltage and a reference value of a break down voltage can be observed.

A warning signal is generated when the measured break down voltage differs to a certain predetermined extend from the reference value.

A warning signal can be an acoustic signal or an electronic signal. The warning signal can be transferred to a remote place to inform a service team.

The measured value of the break down voltage can vary due to statistical effects and thus can differ from the reference value. Thus the value of the break down voltage indicates a good condition of the lubrication, when the measured value is within a certain tolerance of the reference value. Thus there is no need to send out a warning signal when the difference between the measured value and the reference value is within a predetermined range.

Thus the warning signal is only generated when the difference between the measured value and the reference value is significant. Thus the warning signal is only generated when the value of the break down voltage is out of a certain predetermined range.

Thus the warning signal is only generated when the break down voltage low enough, that the condition of the lubrication is not good enough anymore.

Thus the warning signal indicates that service at the bearing is necessary. Thus the warning signal helps to optimize service at the wind turbine.

The warning signal is transmitted to a controller of a wind turbine.

Thus the warning signal is computed in the controller and can be set in relation to other data of the wind turbine.

Thus the controller can slow down or stop the wind turbine when the break down voltage is indicating a bad lubrication in a bearing of the wind turbine. The controller can change the operational mode of the wind turbine to a "low load operation". Thus damage to the bearing is avoided, when the difference between the break down voltage the reference value is bigger then a certain predetermined tolerance.

The controller can also initiate an insertion of lubricant into the bearing to improve the lubrication or an automated exchange of the lubricant in the bearing.

Thus damage in the bearing of the wind turbine is avoided. Thus the life-time of the bearing is increased. Thus the costs for exchange parts and service are minimized.

The first bearing shell is movable in respect to the second bearing shell. The break down voltage is measured at least at two different positions of the first bearing shell in respect to the second bearing shell.

The measurement can be performed while the wind turbine is in operation, thus when the bearing is in operation. The bearing might be rotating when the wind turbine is in operation. For example the main bearing of the wind turbine is in constant operation as soon as the rotor of the wind turbine is rotating.

The measurement can be performed at different times in the ration of the bearing, thus at different positions of the first bearing shell in respect to the second bearing shell.

Thus the measurement reflects different rotational positions of the bearing. Thus a row of measurements of the break down voltage reflects a pattern of values of the break down voltage during the rotation of the bearing.

A bearing can comprise a specific pattern of values of the break down voltage during a rotation. The pattern of the values can be stored and can be compared to measurements later on during operation. Thus a difference in the pattern can be detected. Thus a difference in the pattern can give valuable hints to the condition of the bearing and the lubrication.

Thus the state of the bearing and the lubrication can be monitored with monitoring the break down voltage pattern of the bearing. Thus the service to the wind turbine can be planned to include a special check at the specific bearing. Thus the service can be optimized.

An additional parameter, such as a temperature of the bearing and/or a rotational speed of the bearing and/or a load present in the bearing, is measured. The warning signal is generated in dependency of at least one of the additional parameters.

The value of the break down voltage or the pattern of measurements during the rotation of the bearing can depend on other factors. For example the break down voltage can be higher when the temperature in the bearing is low. Or a measurement the break down voltage can be lower when the load in the bearing is very high.

Thus the measurement can be compared to other factors regarding the wind turbine or the bearing. Thus the value of a measurement can be judged according to other parameters of the wind turbine.

Thus the generation of a warning signal can be done in respect to the other parameters. Thus the generation of a warning signal is optimized. Thus, for example, the wind turbine is not stopped because of one exceptional low value, when at the same time the load in the bearing was very high.

Thus the down-time of the wind turbine is minimized. Thus the cost of energy production is optimized.

The measurement of the break down voltage is performed more frequently when the break down voltage is low.

It can be reasonable to perform the measurement in a certain frequency, for example one measurement in 5 seconds. It can be reasonable to perform the measurement not too often, because the properties of the lubrication might change.

In the case of a measurement of a very low break down voltage a higher frequency of measurement is important to control the condition of the bearing. This can be done by faster increasing the voltage applied to the bearing to build up the potential between the bearing shells faster.

A higher frequency of measurements is also achieved when the rise in the voltage is the same, but the break down voltage is low, so the break down voltage is reached faster. Then the measurement is faster available and the rise in the voltage begins again earlier.

Thus the measurement is performed more frequently when the break down voltage is very low or in a critical range. Thus problems in the bearing or in the lubrication can be monitored better. Thus the control of the bearing is optimized.

A bearing comprises a first bearing shell and a second bearing shell and a layer of lubrication between the first and the second bearing shell.

A bearing further comprises a voltage source with a voltage output to provide a variable voltage, an electrical connection means for the connection of the voltage output of the voltage source to the bearing shells.

A control unit for the control of the voltage level of the voltage source, to apply an increasing electric potential to the bearing shells.

A measurement means for the measurement of the voltage applied to the bearing shell for the detection and measurement of a break-down voltage of a layer of lubrication in the bearing.

Thus the voltage is applied to the bearing and the measurement of the break down voltage can be performed. Thus the value of the break down voltage can be achieved.

A memory unit is connected to the measurement means, to store the measured value of the break down voltage.

Thus the value of the measurement is available for later use, for example to view the development of the bearing over time.

A computational unit is connected to the memory to compare the measured value of the break down voltage and a reference value stored in the memory unit.

Thus the measured value of the break down voltage can be compared with a reference value. Thus the measured value can be evaluated in respect to the reference value. Thus a big difference between a reference value and a measured value can be detected that can point to a problem with the bearing or the lubrication in the bearing.

The computational unit comprises a signal output for a warning signal and/or an error signal.

Thus a warning signal or an error signal can be send from the computational unit to a controller for example. Thus the controller can slow down or stop the wind turbine before the bearing will be damaged. The controller can also send the warning or error signal to a remote service facility where a service for the wind turbine can be planned.

An input is provided at the computational unit for a measurement of additional parameters like a temperature, a rotational speed or a load present in the bearing.

The measured break down voltage depends on different factors that influence the break down voltage of the lubrication and the bearing. For example the break down voltage can vary depending on the temperature of the bearing. The lubrication and thus the break down voltage measured might depend on the rotational speed of the bearing in operation. The break down voltage also depends on the load present in the bearing.

Thus the additional factors can be measured and the values can be used to value the measurements of the break down voltage. It can then be judged whether the break down voltage is within a statistical range of a measurement of a good break down voltage.

Thus real problems in the lubrication can be distinguished from temporary high or low measurements that where influenced by additional factors, but do not point to a problem in the bearing.

Thus warning signals are only produced at measurements that show a real problem in the lubrication. Thus the amount of false warning signal du to measured values that were influenced by other factors then problem can be filtered out.

Thus the amount of warning events is reduced. Thus a too high number of services at the wind turbine is avoided.

The invention is shown in more detail by the help of figures. The figures show a preferred configuration and do not limit the scope of the invention.
- FIG 1: shows an arrangement to monitor the condition of a bearing
- FIG 2: shows a cut through a bearing
- FIG 3: shows the voltage measured at the bearing
- FIG 4: shows the voltage during one revolution of the bearing

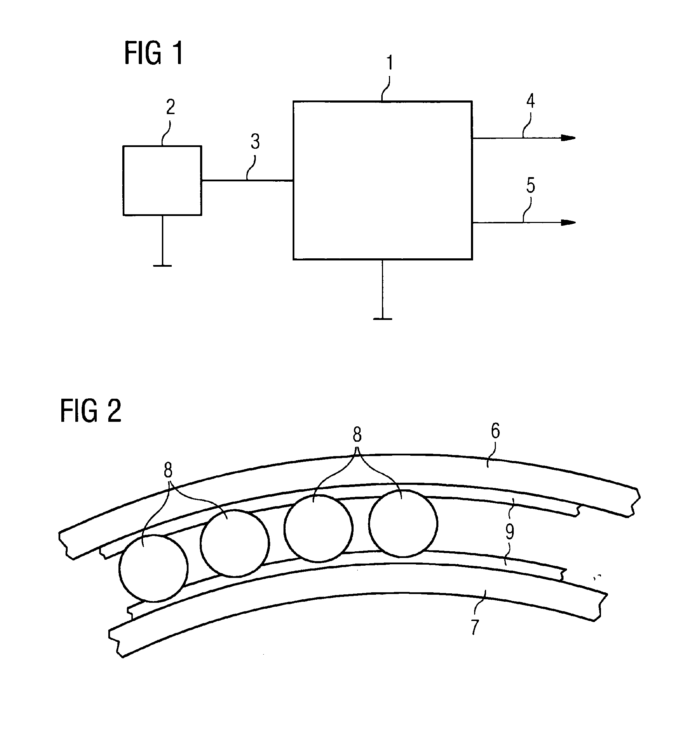

FIG 1 shows an arrangement to monitor the condition of a bearing.

The arrangement 1 to monitor the condition of the bearing 2 comprises a voltage source and a connection 3 to provide a voltage to the bearing 2.

The voltage is applied to one bearing shell of the bearing 2. The other bearing shell can be connected to a common ground within the wind turbine.

The bearing 2 comprises a layer of lubrication between the first and the second bearing shell. The layer of lubrication is to a certain extend insulating, so the first and the second bearing shell act like a capacitor.

The voltage of the voltage source is variable. The voltage can be increased to increase the electric potential between the first and the second bearing shell until the break down voltage is reached. At the break down voltage the electric potential between the first and the second bearing shell will discharge through the layer of lubrication.

The electric potential at the bearing is measured. So the break down voltage can be detected and measured.

The arrangement 1 comprises a memory unit to store the measured value of the break down voltage.

In the memory unit a reference value of the break down voltage of the bearing can be stored. The arrangement comprises a computing unit, where the measured value and the stored reference value are compared.

The arrangement 1 comprises an output 4 to send a warning signal to the controller of the wind turbine. The warning signal can be send, if the break down voltage of the bearing is out of an expected range of measurement values.

The warning signal can also be send, if the break down voltage differs to a certain predetermined extend from the reference value stored in the memory unit.

The arrangement 1 can also send an error signal 5, when the arrangement 1 detects that the measurement is not working properly.

FIG 2 shows a cut through a bearing

FIG 2 shows a cut through a rolling element bearing. The bearing comprises a first bearing shell 6 and a second bearing shell 7. Rolling elements 8 are arranged between the first bearing shell 6 and the second bearing shell 7. Between the bearing shells 6, 7 and the rolling elements 8 there is a layer of lubrication 9.

The layer of lubrication 9 prohibits a metal to metal contact between the bearing shells 6, 7 and the rolling elements 8. The layer of lubrication 9 is at least present between the bearing shells 6, 7 and the rolling elements when the bearing is in operation.

The layer of lubrication 9 is electrically insulating up to a certain break down voltage. The level of the break down voltage depends on the electrical properties of the lubrication and on the thickness of the layer.

The break down voltage is low, when the layer of lubrication is thin or the lubrication contains foreign particles like small metal pieces from abrasion from the bearing.

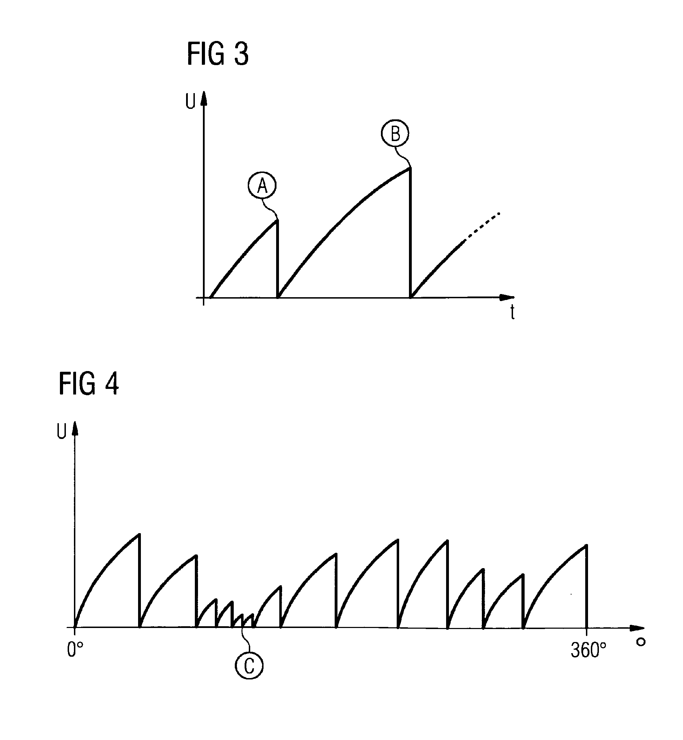

FIG 3 shows the voltage measured at the bearing

FIG 3 shows a diagram of the electric potential applied to the bearing shell over time when the bearing is in operation. The voltage applied to the bearing increases up to the break down voltage. At the break down voltage the potential between the bearing shells discharged. The voltage measured between the bearing shells is then decreasing.

After the discharge the voltage is applied to the bearing shells to build up again a potential between the bearing shells.

This measurement is done during the operation of the bearing. The break down voltage is measured continuously during the revolution of the bearing.

The voltage U at the bearing is increased until a discharge happens. The voltage measured at the time of the discharge is the break down voltage of the bearing at this time. A certain point in time t corresponds to a certain position of the bearing shells in respect to each other.

At point A the break down voltage is low. This is a hint to a thin layer of lubrication or to the presence of foreign particles in the lubrication.

At point B the break down voltage is higher. This shows that the layer of lubrication is good and as a consequence that the lubrication of the bearing at this point is good.

FIG 4 shows the voltage during one revolution of the bearing.

Fig 4 shows the voltage during one complete revolution of the bearing. The diagram shows the voltage U during the revolution of the bearing from an angle of 0° to 360°. At different angles the bearing shows a different break down voltage. This pattern of the break down voltage can be stored in the memory unit of the arrangement to monitor the bearing. It can then be compared with a reference value or with earlier measurement patterns detected.

If the measurements show a very low break down voltage the arrangement can send a warning signal to the wind turbine controller. The controller can then stop the turbine or send a signal to a remote wind turbine control center for example.

In this example there is a very low break down voltage at point C. This could result from a metal to metal contact in the bearing.

The electrical potential between the bearing shells is build up over time.

In a position when the break down voltage is low, the time to build up the potential in the bearing shells that leads to the discharge is short. The break down voltage is reaches in a shorter time.

In a position when the break down voltage is high, the time to build up the potential that leads to the discharge is longer. The break down voltage is reached after a longer time.

In a position with a high break down voltage the measurement is performed less often then in positions when the break down voltage is low.

In a position with a low break down voltage the potential of the discharge is reached faster. Thus leads to a more frequent measurement in an area with a low break down voltage, thus a possible fault.

## Claims

1. Method to measure the condition of the lubrication in a bearing (2) of a wind turbine, whereby the bearing comprises a first bearing shell (6) and a second bearing shell (7) and a layer of lubrication (9) between the first and the second bearing shell (6, 7),
comprising the steps of
- applying a voltage from a voltage source to the bearing shells (6, 7), so that an electrical potential is building up between the bearing shells (6, 7),
- increasing the level of the voltage applied to the bearing shells (6, 7) until the break down voltage of the layer of lubrication (9) is reached,
- measuring the voltage applied to the bearing shells (6, 7) to detect and measure the value of the break-down voltage of the layer of lubrication (9) in the bearing (2).

2. Method according to claim 1, comprising the step of storing the value of the break down voltage in a memory.

3. Method according to claim 2, comprising the step of comparing the measured value of the break down voltage to a reference value.

4. Method according to claim 3, comprising the step of generating a warning signal, when the measured break down voltage differs to a certain predetermined extend from the reference value.

5. Method according to claim 4, comprising the step of transmitting the warning signal to a controller of a wind turbine.

6. Method according to one of the preceding claims, whereby the first bearing shell is movable in respect to the second bearing shell, comprising the step of measuring the break down voltage at least at two different positions of the first bearing shell in respect to the second bearing shell.

7. Method according to one of the claims 4 to 6, whereby an additional parameter such as a temperature of the bearing and/or a rotational speed of the bearing and/or a load present in the bearing is measured, comprising the step of generating the warning signal in dependency of at least one of the additional parameters.

8. Method according to one of the preceding claims, whereby the measurement of the break down voltage is performed more frequently when the break down voltage is low.

9. Arrangement to monitor the condition of lubrication in a bearing of a wind turbine, comprising
- a bearing, whereby the bearing comprises a first bearing shell (6) and a second bearing shell (7) and a layer of lubrication (9) between the first and the second bearing shell,
- a voltage source with a voltage output to provide a variable voltage,
- an electrical connection means (3) for the connection of the voltage output of the voltage source to the bearing shells (6, 7),
- a control unit for the control of the voltage level of the voltage source, to apply an increasing electric potential to the bearing shells (6, 7),
- a measurement means for the measurement of the voltage applied to the bearing shell for the detection and measurement of a break-down voltage of a layer of lubrication in the bearing.

10. Arrangement according to claim 9, comprising a memory unit that is connected to the measurement means, to store the measured value of the break down voltage.

11. Arrangement according to claim 10, comprising a computational unit that is connected to the memory to compare the measured value of the break down voltage and a reference value stored in the memory unit.

12. Arrangement according to claim 11, comprising the computational unit comprises a signal output for a warning signal and/or an error signal.

13. Arrangement according to claim 12, comprising an input to the computational unit for a measurement of additional parameters like a temperature, a rotational speed or a load present in the bearing.

## Patentansprüche

1. Verfahren zum Messen des Zustands der Schmierung in einem Lager (2) einer Windenergieanlage, wobei das Lager eine erste Lagerschale (6) und eine zweite Lagerschale (7) und eine Schicht Schmierung (9) zwischen der ersten und der zweiten Lagerschale (6, 7) umfasst,
mit folgenden Schritten:
- Anlegen einer Spannung aus einer Spannungsquelle an die Lagerschalen (6, 7), so dass sich zwischen den Lagerschalen (6, 7) ein elektrisches Potential aufbaut,
- Erhöhen des an die Lagerschalen (6, 7) angelegten Spannungspegels, bis die Durchschlagsspannung der Schmierungsschicht (9) erreicht ist,
- Messen der an die Lagerschalen (6, 7) angelegten Spannung zum Erkennen und Messen des Werts der Durchschlagsspannung der Schmierungsschicht (9) im Lager (2).

2. Verfahren nach Anspruch 1, das das Speichern des Werts für die Durchschlagsspannung in einem Speicher umfasst.

3. Verfahren nach Anspruch 2, das das Vergleichen des Messwerts für die Durchschlagsspannung mit einem Referenzwert umfasst.

4. Verfahren nach Anspruch 3, das das Erzeugen eines Warnsignals umfasst, wenn sich die gemessene Durchschlagsspannung in einem bestimmten vorgegebenen Ausmaß vom Referenzwert unterscheidet.

5. Verfahren nach Anspruch 4, das das Übermitteln des Warnsignals zu einer Steuerung einer Windenergieanlage umfasst.

6. Verfahren nach einem der vorhergehenden Ansprüche, bei dem die erste Lagerschale in Bezug zur zweiten Lagerschale beweglich ist, mit dem Messen der Durchschlagsspannung an mindestens zwei unterschiedlichen Positionen der ersten Lagerschale in Bezug zur zweiten Lagerschale.

7. Verfahren nach einem der Ansprüche 4 bis 6, bei dem ein zusätzlicher Parameter wie eine Temperatur des Lagers und/ oder eine Drehzahl des Lagers und/ oder eine im Lager vorliegende Last gemessen wird, mit dem Erzeugen des Warnsignals in Abhängigkeit von mindestens einem der zusätzlichen Parameter.

8. Verfahren nach einem der vorhergehenden Ansprüche, bei dem das Messen der Durchschlagsspannung häufiger erfolgt, wenn die Durchschlagsspannung gering ist.

9. Anordnung zum Überwachen des Zustands der Schmierung in einem Lager einer Windenergieanlage mit
- einem Lager, wobei das Lager eine erste Lagerschale (6) und eine zweite Lagerschale (7) und eine Schicht Schmierung (9) zwischen der ersten und der zweiten Lagerschale umfasst,
- einer Spannungsquelle mit einem Spannungsausgang zum Bereitstellen einer variablen Spannung,
- einem elektrischen Verbindungsmittel (3) zum Verbinden des Spannungsausgangs der Spannungsquelle mit den Lagerschalen (6, 7),
- einer Steuereinheit zum Steuern des Spannungspegels der Spannungsquelle für das Anlegen eines zunehmenden elektrischen Potentials an die Lagerschalen (6, 7),
- einem Messmittel zum Messen der an die Lagerschale angelegten Spannung für das Erkennen und Messen einer Durchschlagsspannung einer Schmierungsschicht im Lager.

10. Anordnung nach Anspruch 9, die eine Speichereinheit zum Speichern des Messwerts für die Durchschlagsspannung umfasst, welche mit dem Messmittel verbunden ist.

11. Anordnung nach Anspruch 10, die eine Recheneinheit zum Vergleichen des Messwerts für die Durchschlagsspannung und eines in der Speichereinheit gespeicherten Referenzwerts umfasst, welche mit dem Speicher verbunden ist.

12. Anordnung nach Anspruch 11, die die Recheneinheit umfasst, welche einen Signalausgang für ein Warnsignal und/ oder ein Fehlersignal umfasst.

13. Anordnung nach Anspruch 12, die einen Eingang für die Recheneinheit für eine Messung zusätzlicher Parameter wie einer Temperatur, einer Drehzahl oder einer im Lager vorliegenden Last umfasst.

## Revendications

1. Procédé pour mesurer l'état de la lubrification dans un palier (2) d'une éolienne, dans lequel le palier comprend une première coquille (6) de palier et une deuxième coquille (7) de palier et une couche de lubrification (9) entre la première et la deuxième coquille (6, 7) de palier,
comprenant les étapes de
- application d'une tension provenant d'une source de tension aux coquilles (6, 7) de palier, de telle sorte qu'un potentiel électrique s'accumule entre les coquilles (6, 7) de palier,
- augmentation du niveau de la tension appliquée aux coquilles (6, 7) de palier jusqu'à ce que la tension de rupture de la couche de lubrification (9) soit atteinte,
- mesure de la tension appliquée aux coquilles (6, 7) de palier pour détecter et mesurer la valeur de la tension de rupture de la couche de lubrification (9) dans le palier (2).

2. Procédé selon la revendication 1, comprenant l'étape de stockage de la valeur de la tension de rupture dans une mémoire.

3. Procédé selon la revendication 2, comprenant l'étape de comparaison de la valeur mesurée de la tension de rupture à une valeur de référence.

4. Procédé selon la revendication 3, comprenant l'étape de génération d'un signal d'avertissement, lorsque la tension de rupture mesurée diffère dans une certaine mesure prédéterminée de la valeur de référence.

5. Procédé selon la revendication 4, comprenant l'étape de transmission du signal d'avertissement à un contrôleur d'une éolienne.

6. Procédé selon l'une des revendications précédentes, dans lequel la première coquille de palier est mobile par rapport à la deuxième coquille de palier, comprenant l'étape de mesure de la tension de rupture au moins en deux positions différentes de la première coquille de palier par rapport à la deuxième coquille de palier.

7. Procédé selon l'une des revendications 4 à 6, dans lequel un paramètre additionnel tel qu'une température du palier et/ou une vitesse de rotation du palier et/ou une charge présente dans le palier est mesuré, comprenant l'étape de génération d'un signal d'avertissement en fonction d'au moins un des paramètres additionnels.

8. Procédé selon l'une des revendications précédentes, dans lequel la mesure de la tension de rupture est effectuée plus fréquemment lorsque la tension de rupture est basse.

9. Agencement pour surveiller l'état de lubrification dans un palier d'une éolienne, comprenant
- un palier, dans lequel le palier comprend une première coquille (6) de palier et une deuxième coquille (7) de palier et une couche de lubrification (9) entre la première et la deuxième coquille de palier,
- une source de tension avec une sortie de tension pour délivrer une tension variable,
- un moyen (3) de connexion électrique pour la connexion de la sortie de tension de la source de tension aux coquilles (6, 7) de palier,
- une unité de commande pour la commande du niveau de tension de la source de tension, pour appliquer un potentiel électrique croissant aux coquilles (6, 7) de palier,
- un moyen de mesure pour la mesure de la tension appliquée à la coquille de palier pour la détection et la mesure d'une tension de rupture d'une couche de lubrification dans le palier.

10. Agencement selon la revendication 9, comprenant une unité de mémoire qui est connectée au moyen de mesure, pour stocker la valeur mesurée de la tension de rupture.

11. Agencement selon la revendication 10, comprenant une unité de calcul qui est connectée à la mémoire pour comparer la valeur mesurée de la tension de rupture et une valeur de référence stockée dans l'unité de mémoire.

12. Agencement selon la revendication 11, dans lequel l'unité de calcul comprend une sortie de signal pour un signal d'avertissement et/ou un signal d'erreur.

13. Agencement selon la revendication 12, comprenant une entrée dans l'unité de calcul pour une mesure de paramètres additionnels tels qu'une température, une vitesse de rotation ou une charge présente dans le palier.
